# FASCICULE DE BREVET EUROPEEN

(11) **EP 1 139 935 B1**
(45) Date de publication et mention de la délivrance du brevet: **09.05.2007**
(21) Numéro de dépôt: 99958285.1
(22) Date de dépôt: 09.12.1999
(51) Int. Cl.: A61F 2/44

(54) **PROTHESE DE DISQUE INTERVERTEBRAL A COMPORTEMENT MECANIQUE AMELIORE**
BANDSCHEIBENPROTHESE MIT VERBESSERTEM MECHANISCHEN VERHALTEN
INTERVERTEBRAL DISC PROSTHESIS WITH IMPROVED MECHANICAL BEHAVIOUR

(30) Priorité: 11.12.1998 FR 9815672
(43) Date de publication de la demande: 10.10.2001
(73) Titulaire: Stryker Spine, 33610 Cestas (FR)
(72) Inventeur: GAUCHET, Fabien, 60800 Duvy (FR); LE COUEDIC, Régis, 33610 Cestas (FR)
(74) Mandataire: Le Forestier, Eric
(86) Numéro de dépôt international: PCT/FR1999/003073
(87) Numéro de publication internationale: WO 2000/035385

(56) Documents cités:
- EP-A- 0 277 282
- DE-A- 2 263 842
- DE-U- 9 000 094
- FR-A- 2 723 841

## Description

L'invention concerne les prothèses de disque intervertébral.

On connaît d'après le document EP-0 356 112 une prothèse de disque intervertébral comportant deux plateaux et un coussin en matériau compressible interposé entre les plateaux et fixé à ceux-ci. Une telle prothèse autorise une importante mobilité entre les deux vertèbres adjacentes. Elle peut notamment subir des mouvements de compression entre les deux vertèbres, et de torsion suivant trois axes perpendiculaires entre eux. Son comportement mécanique est donc proche de celui d'un disque intervertébral naturel sain. Toutefois, elle a notamment pour inconvénient que les sollicitations latérales de chaque vertèbre sur le disque suivant une direction perpendiculaire à la direction longitudinale du rachis sont transmises en intégralité à l'autre vertèbre, ce qui n'est pas le cas dans un vrai disque sain. Or, ces contraintes de cisaillement sont nuisibles.

Le document DE-90 00 034-U divulgue une prothèse conforme au préambule de la revendication 1. Le document DE-22 63 842 divulgue une prothèse comportant deux plateaux et un coussin.

Un but de l'invention est de fournir une prothèse de disque d'un type différent et permettant d'approcher encore davantage les propriétés mécaniques d'un disque intervertébral naturel sain.

En vue de la réalisation de ce but, on prévoit selon l'invention une prothèse conforme à la revendication 1.

Ainsi, on évite l'apparition de sollicitations latérales trop importantes entre les deux vertèbres suivant une direction perpendiculaire à la direction longitudinale du rachis. On reproduit donc mieux le comportement d'un disque naturel sain.

Avantageusement, la ou chaque extrémité du corps est logée dans un renfoncement de l'un des plateaux apte à former une butée latérale pour cette extrémité.

Ainsi, on peut limiter les déplacements relatifs latéraux entre le corps et le ou les plateaux.

Avantageusement, la ou chaque extrémité présente une face ayant une zone de contact avec une face du plateau associé, les faces étant agencées de sorte que la zone de contact a une superficie qui augmente lorsqu'on augmente une sollicitation du plateau en direction du corps.

Ainsi, pour les valeurs de compression les plus basses, la réaction mécanique de la prothèse lors de la compression du corps varie très peu en fonction du changement de dimension du corps suivant la direction de compression. Autrement dit, la courbe de cette réaction en fonction de la variation de hauteur du coussin est peu inclinée par rapport à l'horizontale pour de faibles valeurs de compression et on fournit peu d'effort en début de course. Cette propriété reproduit celle d'un disque naturel sain.

Avantageusement, la ou chaque extrémité présente une face de contact avec une face du plateau associé, les deux faces étant courbes dans au moins une direction commune et étant respectivement concave et convexe.

Ainsi, après un déplacement latéral relatif du corps et du plateau, ces deux faces assurent un auto-centrage de ces éléments les replaçant dans une disposition coaxiale. Lorsque la face concave a au moins un rayon de courbure supérieur à un rayon de courbure correspondant de la face convexe, on obtient en outre une zone de contact de superficie variable telle que précitée. -

Avantageusement, le corps comprend un matériau viscoélastique, notamment du silicone.

Ainsi, ce matériau a, en compression, un comportement tel que la courbe précitée a une forme en hystérésis, ce qui rapproche encore le comportement de la prothèse de celui du disque naturel sain.

Avantageusement, la prothèse comprend un fluide interposé entre les plateaux.

Le fluide, notamment lorsqu'il est compressible, accroît encore la forme en hystérésis.

Avantageusement, le fluide est en contact avec les plateaux.

Avantageusement, le fluide s'étend en périphérie du corps.

Avantageusement, la prothèse comporte une enceinte renfermant le fluide et agencée de sorte qu'elle a une superficie de section transversale parallèlement aux plateaux sensiblement invariable lorsque varie une sollicitation des plateaux l'un vers l'autre.

Avantageusement, la prothèse est destinée à la zone lombaire du rachis.

D'autres caractéristiques et avantages de l'invention apparaîtront encore dans la description suivante d'un mode préféré de réalisation et de deux variantes donnés à titre d'exemples non limitatifs. Aux dessins annexés :
- la figure 1 est une vue en perspective d'une prothèse selon l'invention ;
- la figure 2 est une vue en coupe axiale selon le plan II-II de la prothèse de la figure 1 ;
- la figure 3 est une vue à échelle agrandie d'un détail D de la figure 2 ;
- la figure 4 est une courbe indiquant la force de compression F exercée par les deux plateaux sur le coussin en fonction de la variation de la distance les séparant ;
- la figure 5 est une vue en coupe d'un détail d'une variante de réalisation de la prothèse ; et
- la figure 6 est une vue simplifiée analogue à la figure 2 montrant une deuxième variante de réalisation.

La prothèse de disque intervertébral 2 selon l'invention est ici particulièrement destinée à la zone lombaire de la colonne vertébrale du corps humain. Elle comporte deux plateaux plats 4 ayant une forme générale en haricot à hile postérieur en vue en plan. Chaque plateau 4 comporte une plaque circulaire centrale 6 et une couronne 8 s'étendant en périphérie de la plaque dans le plan de celle-ci. Au repos, les deux plateaux 4 s'étendent parallèlement l'un à l'autre, à distance et en regard l'un de l'autre avec leurs contours en coïncidence. Sur chaque plateau 4, la couronne 8 et la plaque 6 présentent chacune une gorge 27 pour la réception d'un joint 31.

La prothèse de disque 2 comporte un coussin ou partie intermédiaire 10 interposé entre les deux plateaux 4. Le coussin comporte un corps solide compressible 12, ici en matériau viscoélastique, par exemple en silicone. Ce corps a une dureté shore-A avantageusement comprise entre 60 et 100, et ici d'environ 80. Le corps 12 a une forme de révolution autour de son axe principal 14. Il présente une face latérale cylindrique 16 et deux faces d'extrémités axiales 18 généralement perpendiculaires à l'axe 14 et de forme légèrement sphérique convexe. Chaque face 18 présente donc deux courbures identiques dans des plans perpendiculaires entre eux. Le corps 12 est disposé coaxialement avec les plaques 6. Chaque plaque 6 présente une face centrale interne plane 20 perpendiculaire à l'axe 14 et en contact avec une des extrémités axiales 18 respectives du corps 12. Ainsi, la face sphérique convexe 18 du corps est en appui sur la face plane 20 du plateau. Le corps 12 est en appui sans ancrage sur chacun des plateaux 4 de sorte qu'il est mobile par rapport à chacun de ces plateaux suivant une direction parallèle aux plateaux, c'est-à-dire perpendiculaire à l'axe principal 14. Compte tenu de la compression du corps 12 exercée par les plateaux 4, et compte tenu de la forme des faces des plateaux et du corps, la mobilité suivant cette direction se traduit par un roulement, éventuellement sans glissement, de chaque extrémité axiale 18 du corps sur la face 20 du plateau avec laquelle elle est en contact. Le corps 12 roule donc entre les deux plateaux. Ceux-ci se déplacent alors latéralement l'un par rapport à= l'autre tout en demeurant si besoin parallèles entre eux. On évite ainsi la transmission de contraintes latérales de l'une à l'autre des vertèbres.

Le coussin 10 comporte en outre un soufflet 22. Le soufflet entoure le corps 14 coaxialement à celui-ci et à distance de celui-ci. Il a une forme symétrique de révolution autour de l'axe 14. Sa paroi présente de profil des ondulations 24 permettant de faire varier la longueur du soufflet 22 suivant la direction axiale 14, sans que varie sensiblement la superficie de sa section transversalement à l'axe 14. En l'espèce, ce soufflet, de même que les plateaux 4, est réalisé en titane ou alliage de titane, de sorte qu'il présente une certaine rigidité axiale et forme un ressort de compression. Il peut également être déformé suivant une direction perpendiculaire à l'axe 14 ou subir une torsion autour de l'axe 14 ou d'un axe quelconque perpendiculaire à celui-ci.

Le soufflet 22 présente à ses deux extrémités axiales des bords collés à des bords respectifs des plaques 6 s'étendant en saillie de la face interne 20. Le collage est réalisé de façon étanche de sorte que le soufflet 22 définit avec les deux plaques 6 une enceinte étanche à volume variable s'étendant autour du corps 12. Cette enceinte renferme un fluide, en l'espèce un gaz qui est ici de l'air. Les ondulations 24 les plus proches du corps 12 s'étendent à distance de celui-ci pour permettre une libre circulation du gaz de l'une à l'autre des plaques 6.

Le soufflet 22 présente en l'espèce dix convolutions, soit huit crêtes externes en plus des deux crêtes de fixation aux plateaux. Il a ici un diamètre externe d'environ 30 mm et un diamètre interne d'environ 17 mm. Sa hauteur, lorsque la prothèse est hors charge, vaut 10 mm. La paroi du soufflet peut être réalisée au moyen d'une, deux ou trois feuilles chacune de 0,1 mm d'épaisseur et dont la somme des épaisseurs forme l'épaisseur de la paroi. Le soufflet a ici en propre une raideur d'environ 1,6 N/mm.

Chaque couronne 8 comporte deux pattes 25 s'étendant en saillie d'une face externe du plateau 4 perpendiculairement au plan du plateau. Chaque patte 25 présente un orifice 27 la traversant de part en part en direction du centre de la plaque et, sur une face de la patte 25 opposée au plateau 4, une empreinte de forme sphérique. Les orifices 27 permettent la réception d'une vis à os 26 ayant une tête 28 dont une face inférieure a une forme sphérique mâle coopérant avec l'empreinte femelle de la patte 25 pour permettre une libre orientation de la vis 26 par rapport à la patte associée.

Pour réaliser un ancrage à court terme de la prothèse de disque 2 dans la colonne, on pourra ancrer les vis 26 dans le spondyle des vertèbres adjacentes au disque à remplacer.

Toutefois, on pourra prévoir un ancrage dit à long terme où, en outre, les surfaces des plateaux 4 en contact avec les vertèbres adjacentes sont recouvertes d'hydroxyapatite, ou de toute autre substance connue en soi pouvant stimuler la croissance osseuse. Avant recouvrement, lesdites surfaces pourront être traitées pour obtenir un état de surface plus ou moins poreux, présentant des points d'ancrage pour le tissu osseux, pour assurer une meilleure interface avec ledit tissu osseux.

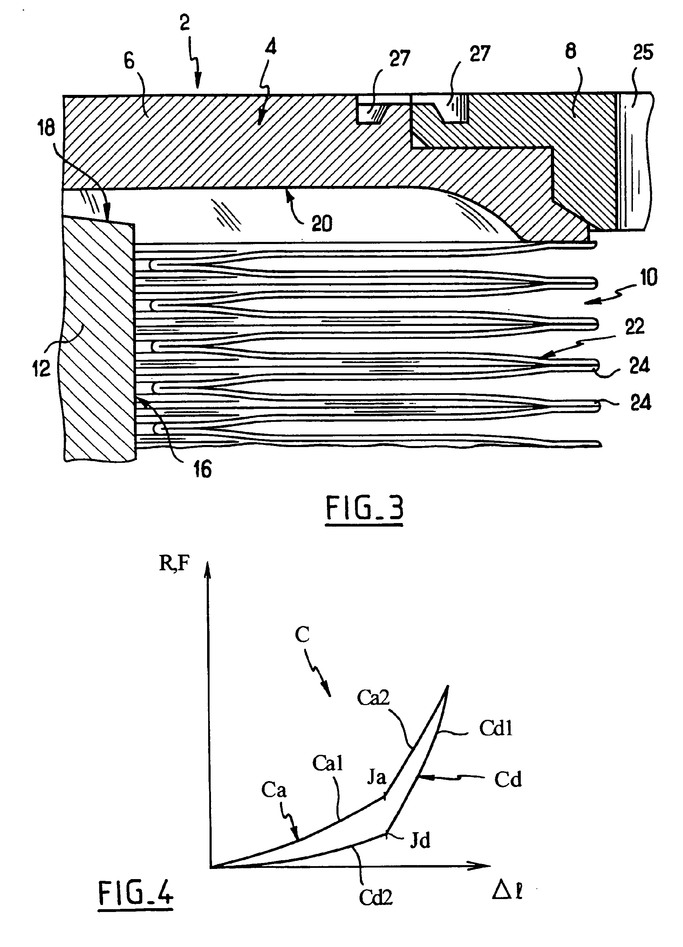

On a représenté en figure 4 l'allure de la courbe C indiquant l'intensité d'un effort de compression F exercé sur le coussin 10 (c'est-à-di're sur les deux plateaux 4) en faisant abstraction de leur déformabilité, quasi nulle, suivant la direction axiale 14, en fonction de la variation de la longueur 1 du coussin suivant la direction axiale 14 (ou encore de la distance entre les deux plateaux). Cette courbe représente également la réaction mécanique R du coussin 10 dans les mêmes conditions. Cette courbe C n'est pas linéaire. De plus, elle présente une forme en hystérésis : la courbe Ca indiquant l'augmentation de la compression F à partir de l'origine zéro étant distincte de celle Cd indiquant la diminution de la compression F jusqu'à l'origine, et s'étendant tout entière au-dessus de cette dernière. Cette forme en hystérésis prononcée est due principalement au matériau viscoélastique du corps et subsidiairement à l'association dans le coussin 10 du corps 12 et du fluide.

En outre, la courbe Ca, relative à l'augmentation de la force de compression F, présente- à partir de l'origine O une portion Ca1 à faible pente, puis une portion Ca2 à pente plus forte. La courbe Cd illustrant la diminution de la compression F présente pour les valeurs les plus élevées de la force F une portion Cd1 de forte pente, puis pour les valeurs les plus basses de la force F une portion Cd2 de pente plus faible. La présence d'une portion de faible pente au voisinage de l'origine pour les courbes Ca et Cd est due principalement à la conformation des faces de contact 18, 20 du corps 12 et des plateaux 4, qui entraîne que la superficie de la zone de contact mutuel entre chaque plateau et le corps, généralement en forme de disque, augmente lorsqu'on augmente la force F. Cette augmentation se produit jusqu'à atteindre la superficie maximale de la zone de contact, lorsque toute la face 18 touche le plateau 4.

Les points de raccordement Ja et Jd forment respectivement la jonction entre les courbes Ca1 et Ca2, et Cd1 et Cd2. Sur la courbe Ca, le point Ja correspond à l'effort F pour lequel les surfaces maximales de contact entre les plateaux et le corps sont atteintes. De même, sur la courbe Cd, le point Jd correspond à l'effort pour lequel ces surfaces cessent d'être maximales.

La prothèse pourra être configurée de sorte que le point Ja corresponde à une valeur de Δl située entre 25% et 75% de la variation maximale de longueur envisagée pour la prothèse en utilisation.

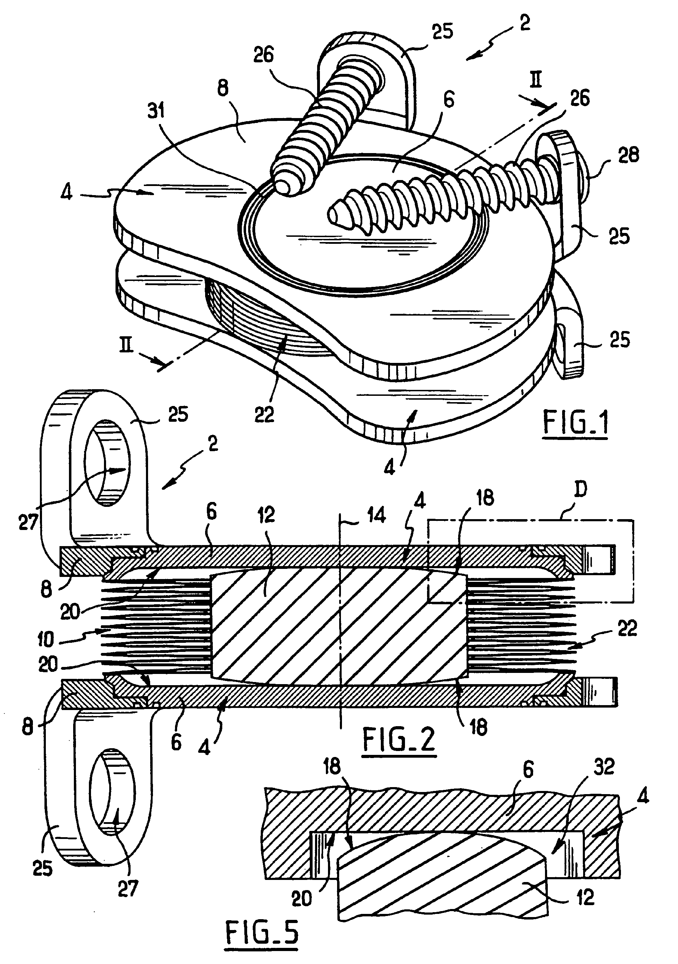

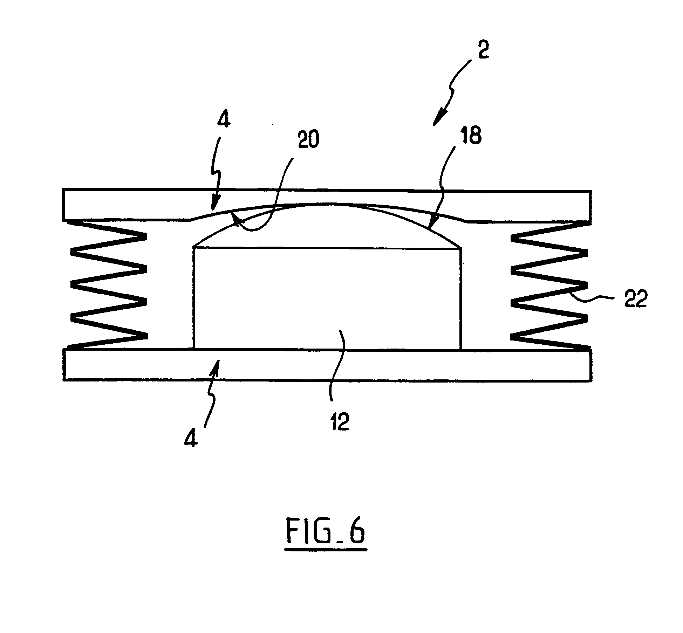

En référence à la figure 5, on pourra prévoir dans une variante de réalisation (présentant par ailleurs les autres caractéristiques de la prothèse de la figure 1) que la face 20 de chaque plateau 4 en regard du corps 12 présente un renfoncement 32, ici en « U », formant butée latérale, dans lequel vient se loger l'extrémité axiale 18 correspondante du corps. On limite ainsi à une certaine plage les déplacements relatifs latéraux du corps 12 par rapport à chaque plateau 4.

Dans la variante de la figure 6, la face 20 peut être courbe et concave dans une ou deux directions, comme c'est le cas ici, et la face 18 peut être courbe et convexe dans la ou les directions correspondantes, le rayon de courbure de la face 20 étant, pour chaque direction, plus grand que celui de la face 18 dans la direction correspondante. Les deux faces 18, 20 sont ici sphériques. Les rayons de courbure des surfaces 18 et 20 seront par exemple compris entre 70 et 80 mm, et 140 et 200 mm respectivement. Un tel agencement permet d'obtenir un auto-centrage des deux faces tout en autorisant un déplacement latéral relatif du corps 12 par rapport au plateau suivant une direction quelconque perpendiculaire à une direction longitudinale du rachis.

Dans le mode de réalisation de la figure 2, les deux extrémités du corps 12 présentent une surface de contact 18 avec le plateau associé de superficie variable et le rendant mobile latéralement par rapport au corps.

Au contraire, dans la variante de la figure 6, seule l'une des extrémités 18 du corps 12 présente cette propriété. L'autre extrémité, inférieure sur la figure 6, a une forme plane circulaire à zone de contact invariable avec le plateau associé et fixe par rapport à celui-ci.

Bien entendu, on pourra apporter à l'invention de nombreuses modifications sans sortir du cadre de celle-ci.

Le fluide pourra être un liquide, voire un mélange d'un liquide et d'un gaz, ce dernier étant par exemple faiblement soluble dans le liquide.

Le corps pourra avoir une forme elliptique en section transversale à l'axe 14.

La courbure des faces pourra être limitée à un seul plan.

On pourra mettre en oeuvre les caractéristiques relatives à l'enveloppe 22 (ressort, distance au corps 12) indépendamment des autres caractéristiques.

## Revendications

1. Prothèse de disque intervertébral comportant deux plateaux (4) et un coussin (10) interposé entre les plateaux, le coussin comportant un corps compressible (12) présentant des extrémités (18) en contact avec des faces en regard des plateaux (4), où au moins l'une des extrémités (18) est libre de se déplacer par rapport au plateau associé suivant une direction parallèle au plateau, prothèse **caractérisée en ce qu'**au moins l'une desdites extrémités (18) est convexe et présente un rayon de courbure inférieur au rayon de courbure de la face en regard (20), concave, du plateau associé.

2. Prothèse selon la revendication 1, **caractérisée en ce que** la ou chaque extrémité (18) du corps est logée dans un renfoncement (32) de l'un des plateaux (4) apte à former une butée latérale pour cette extrémité.

3. Prothèse selon la revendication 1 ou 2, **caractérisée en ce que** la ou chaque extrémité présente une face (18) ayant une zone de contact avec une face (20) du plateau associé, les faces (18,20) étant agencées de sorte que la zone de contact a une superficie qui augmente lorsqu'on augmente une sollicitation du plateau (4) en direction du corps (12).

4. Prothèse selon l'une quelconque des revendications 1 à 3, **caractérisée en ce que** le corps (12) comprend un matériau viscoélastique.

5. Prothèse selon la revendication 4, **caractérisée en ce que** le matériau viscoélastique est du silicone.

6. Prothèse selon l'une quelconque des revendications 1 à 5, **caractérisée en ce qu'**elle comprend un fluide interposé entre les plateaux (4).

7. Prothèse selon la revendication 6, **caractérisée en ce que** le fluide est en contact avec les plateaux (4).

8. Prothèse selon la revendication 6 ou 7, **caractérisée en ce que** le fluide s'étend en périphérie du corps (12).

9. Prothèse selon l'une quelconque des revendications 6 à 8, **caractérisée en ce qu'**elle comporte une enceinte (22) renfermant le fluide et agencée de sorte qu'elle a une superficie de section transversale parallèlement aux plateaux (4) sensiblement invariable lorsque varie une sollicitation des plateaux l'un vers l'autre.

10. Prothèse selon l'une quelconque des revendications 1 à 9, **caractérisée en ce qu'**il s'agit d'une prothèse de disque intervertébral lombaire.

## Claims

1. Intervertebral disc prosthesis comprising two plates (4) and a cushion (10) interposed between the plates, the cushion comprising a compressible body (12) which has ends (18) in contact with opposite faces of the plates (4), where at least one of the ends (18) is freely displaceable relative to the associated plate in a direction parallel to the plate, which prosthesis is **characterized in that** at least one of said ends (18) is convex and has a radius of curvature smaller than the radius of curvature of the concave opposite face (20) of the associated plate.

2. Prosthesis according to Claim 1, **characterized in that** the end or each end (18) of the body is lodged in a recess (32) which is provided in one of the plates (4) and which can form a lateral abutment for this end.

3. Prosthesis according to Claim 1 or 2, **characterized in that** the end or each end has a face (18) having a zone of contact with a face (20) of the associated plate, said faces (18, 20) being arranged in such a way that the zone of contact has a surface area which increases when a stressing of the plate (4) in the direction of the body (12) increases.

4. Prosthesis according to any one of Claims 1 to 3, **characterized in that** the body (12) comprises a viscoelastic material.

5. Prosthesis according to Claim 4, **characterized in that** the viscoelastic material is silicone.

6. Prosthesis according to any one of Claims 1 to 5, **characterized in that** it comprises a fluid interposed between the plates (4).

7. Prosthesis according to Claim 6, **characterized in that** the fluid is in contact with the plates (4).

8. Prosthesis according to Claim 6 or 7, **characterized in that** the fluid extends about the periphery of the body (12).

9. Prosthesis according to any one of Claims 6 to 8, **characterized in that** it comprises a chamber (22) enclosing the fluid and arranged in such a way that it has a surface area of transverse section parallel to the plates (4) which is substantially invariable when a stress moving the plates toward one another varies.

10. Prosthesis according to any one of Claims 1 to 9, **characterized in that** it is an intervertebral lumbar disc prosthesis.

## Patentansprüche

1. Bandscheibenprothese, die zwei Platten (4) und ein zwischen den Platten angeordnetes Polster (10) aufweist, wobei das Polster einen komprimierbaren Körper (12) mit zwei Endabschnitten (18) in Kontakt mit gegenüberliegenden Flächen der Platten (4) enthält, wobei mindestens der eine der Endabschnitte (18) bezüglich der zugehörigen Platte in einer Richtung parallel zu der Platte frei beweglich ist, wobei die Prothese
**dadurch gekennzeichnet ist, dass**
mindestens einer der Endabschnitte (18) konvex ist und einen Krümmungsradius aufweist, der kleiner ist als der Krümmungsradius der gegenüberliegenden konkaven Fläche (20) der zugehörigen Platte.

2. Prothese nach Anspruch 1,
**dadurch gekennzeichnet ist, dass**
der oder die Endabschnitte (18) des Körpers in eine Verstärkung (32) einer der Platten (4) eingesetzt sind, die einen seitlichen Anschlag für diese Endabschnitte bilden können.

3. Prothese nach Anspruch 1 oder 2,
**dadurch gekennzeichnet ist, dass**
der oder die Endabschnitte (18) eine Fläche (18) aufweisen, die einen Kontakbereich mit einer Fläche (20) der zugehörigen Platte besitzt, wobei diese Flächen (18, 20) so ausgestaltet sind, dass der Kontaktbereich eine Oberfläche aufweist, die sich vergrößert, wenn sich die Belastung der Platte (4) in Richtung des Körpers (12) vergrößert.

4. Prothese nach einem der Ansprüche 1 bis 3,
**dadurch gekennzeichnet ist, dass**
der Körper (12) ein viskoelastisches Material enthält.

5. Prothese nach Anspruch 4,
**dadurch gekennzeichnet ist, dass**
das viskoelastische Material Silikon ist.

6. Prothese nach einem der Ansprüche 1 bis 5,
**dadurch gekennzeichnet ist, dass**
sie ein zwischen den Platten (4) angeordnetes Fluid enthält.

7. Prothese nach Anspruch 6,
**dadurch gekennzeichnet ist, dass**
das Fluid mit den Platten (4) in Kontakt steht.

8. Prothese nach Anspruch 6 oder 7,
**dadurch gekennzeichnet ist, dass**
das Fluid über den Umfang des Körpers (12) verteilt ist.

9. Prothese nach Anspruch 6 bis 8,
**dadurch gekennzeichnet ist, dass**
sie eine Einfassung (22) aufweist, welche das Fluid einschliesst und die so ausgelegt ist, dass sie eine Oberfläche mit einem Querschnitt bildet, der parallel zu den Platten (4) liegt und weitgehend unverändert bleibt, wenn eine Belastung der Platten untereinander verändert wird.

10. Prothese nach einem der Ansprüche 1 bis 9,
**dadurch gekennzeichnet ist, dass**
es sich um eine Lendenwirbel-Bandscheibenprothese handelt.
